# EUROPÄISCHE PATENTSCHRIFT

(11) **EP 3 197 858 B1**
(45) Veröffentlichungstag und Bekanntmachung des Hinweises auf die Patenterteilung: **06.02.2019**
(21) Anmeldenummer: 15766172.9
(22) Anmeldetag: 21.09.2015
(51) Int. Cl.: C07C 67/08, C07C 69/82

(54) **VERFAHREN ZUR HERSTELLUNG VON TEREPHTHALSÄUREDIESTERN MIT UMWÄLZUNG DES REAKTIONSGEMISCHES**
METHOD FOR PRODUCING DIESTERS OF TEREPHTHALIC ACID WITH CIRCULATION OF THE REACTION MIXTURE
PROCÉDÉ DE PRODUCTION DE DIESTERS D'ACIDE TÉRÉPHTALIQUE AVEC RECIRCULATION DU MÉLANGE RÉACTIONNEL

(30) Priorität: 24.09.2014 EP 14186141
(43) Veröffentlichungstag der Anmeldung: 02.08.2017
(73) Patentinhaber: BASF SE, 67056 Ludwigshafen (DE)
(72) Erfinder: SCHRAUT, Armin, 64625 Bensheim (DE); KALLER, Martin, 68163 Mannheim (DE); BRONNEBERG, Rob, 67319 Wattenheim (DE); STAMMER, Jasmin, 67251 Freinsheim (DE); DAS, Martin, 68163 Mannheim (DE); HARNISCHMACHER, Gerrit, 68159 Mannheim (DE)
(74) Vertreter: BASF IP Association
(86) Internationale Anmeldenummer: PCT/EP2015/071574
(87) Internationale Veröffentlichungsnummer: WO 2016/046117

(56) Entgegenhaltungen:
- EP-A2- 1 186 593
- JP-A- 2007 077 041
- US-A- 2 825 738
- US-A- 3 927 982

## Beschreibung

Die Erfindung betrifft ein Verfahren zur Herstellung von Terephthalsäurediestern durch Umsetzung von Terephthalsäure mit wenigstens einem Alkohol.

Ester der Terephthalsäure finden Anwendung als Weichmacher und zeichnen sich durch günstige toxikologische Eigenschaften aus.

Es ist bekannt, Carbonsäureester durch Umsetzung von Carbonsäuren mit Alkoholen herzustellen. Diese Reaktion kann autokatalytisch oder katalytisch, beispielsweise durch Brönstedt- oder Lewissäuren, durchgeführt werden. Unabhängig von der Art der Katalyse entsteht immer ein temperaturabhängiges Gleichgewicht zwischen den Einsatzstoffen (Carbonsäure und Alkohol) und den Produkten (Ester und Wasser).

Um das Gleichgewicht zu Gunsten des Esters (bzw. des Vollesters bei mehrbasigen Säuren) zu verschieben, wird in der Regel ein Schleppmittel eingesetzt, mit dessen Hilfe das Reaktionswasser aus dem Ansatz entfernt wird. Wenn einer der Einsatzstoffe (Alkohol oder Carbonsäure) niedriger siedet als der gebildete Ester und mit Wasser eine Mischungslücke bildet, kann ein Edukt als Schleppmittel verwendet und nach Abtrennung von Wasser wieder in den Ansatz zurückgeführt werden. Bei der Veresterung von höheren aliphatischen Carbonsäuren, aromatischen Carbonsäuren oder von zwei- oder mehrbasigen Carbonsäuren ist in der Regel der eingesetzte Alkohol das Schleppmittel.

Dient der eingesetzte Alkohol als Schleppmittel, geht man üblicherweise so vor, dass man den Brüden aus dem Reaktor zumindest teilweise kondensiert, das Kondensat in eine wässrige Phase und eine im Wesentlichen aus dem zur Veresterung eingesetzten Alkohol bestehende organische Phase trennt und die organische Phase zumindest teilweise in den Reaktor zurückführt.

Die EP-A 1 186 593 beschreibt ein Verfahren zur Herstellung von Carbonsäureestern durch Reaktion von Di- oder Polycarbonsäuren oder deren Anhydriden mit Alkoholen, wobei das Reaktionswasser durch azeotrope Destillation mit dem Alkohol entfernt wird. Die durch die azeotrope Destillation aus der Reaktion entfernte Flüssigkeitsmenge wird vollständig oder teilweise mit dem Alkohol wieder ergänzt.

Die WO 2010/076192 A1 schlägt vor, Leichtsieder aus der zurückzuführenden organischen Phase zu entfernen, um deren Anreicherung im Reaktorsystem zu verhindern.
Die US 7,276,621 B2 beschreibt ein Verfahren zur Titanat-katalysierten Veresterung von Terephthalsäure mit 2-Ethylhexanol. Ein Inertgas wird durch die Reaktionsmischung geleitet, um die Entfernung von Wasser zu unterstützen.
Auch die JP 4956945 B2 beschreibt ein Verfahren zur Veresterung von Terephthalsäure mit 2-Ethylhexanol. Die Terephthalsäure wird dabei als Aufschlämmung kontinuierlich oder diskontinuierlich in das Reaktionssystem eingebracht. Die Zudosierung erfolgt dabei in der gleichen Geschwindigkeit, in der die Terephthalsäure zum Produkt umgesetzt wird.
Die US 7,799,942 B2 beschreibt ein Verfahren zur Herstellung von Terephthalsäurediestern in einem Reaktor bei Atmosphärendruck unter Verwendung einer auf den Reaktor aufgesetzten Destillationskolonne. Zusätzlich wird die Reaktionsmischung von einem Inertgas durchströmt.
Die WO 2010/076193 A1 beschreibt ein Verfahren zur Aufreinigung des rohen Esterproduktes einer Veresterungsreaktion, bei der ein metallhaltiger Veresterungskatalysator eingesetzt wird.

US 2,825,738 offenbart die Veresterung einer Suspension von Terephthalsäure mit Methanol, in welchem die Mischung von Methanol und Wasser abdestilliert und getrennt wird. Der so erhaltene wasserfreies Methanol wird in das Reaktionssystem zurückgeführt.

Die Löslichkeit von Terephthalsäure in höheren Alkoholen ist gering. Beispielsweise ist Terephthalsäure in 2-Ethylhexanol bei 180 °C nur zu weniger als 0,65 Gew.-% löslich.

Die Umsetzung von Terephthalsäure mit höheren Alkoholen verläuft nur über den Teil an Terephthalsäure, der im Alkohol gelöst vorliegt. Für das Erreichen hoher Umsätze ist es unerlässlich, eine konstante Durchmischung des heterogenen Gemisches von Terephthalsäure und Alkohol und einen effektiven Wärmeeintrag in das Reaktionssystem zu gewährleisten. Weiterhin ist es wichtig den Wassergehalt in der Reaktionsmischung gering zu halten, um das Reaktionsgleichgewicht auf die Produktseite verschieben zu können und, falls hydrolyseempfindliche Veresterungskatalysatoren eingesetzt werden, die Hydrolyse des Katalysators zu verhindern. Die Zudosierung fester Terephthalsäure in den Reaktor mit siedendem Alkohol, z.B. über eine Förderschnecke, bei der das Pulver am freien Ende der Schnecke in freiem Fall in den Reaktor fällt, ist wegen der Gefahr des Verklumpens der Terephthalsäure nur mit Schwierigkeiten möglich. Bei hohen Reaktoren großer Volumina ist das Anbringen eines Vorratsbehälters für Terephthalsäure oberhalb des Reaktors oft mit baulichen Schwierigkeiten verbunden.

Der Erfindung liegt daher die Aufgabe zugrunde, ein Verfahren zur Herstellung von Terephthalsäurediestern bereitzustellen, das einen einfachen Eintrag der Terephthalsäure in den Reaktor erlaubt, eine effektive Durchmischung der Reaktionsmischung und einen effektiven Wärmeeintrag in das Reaktionssystem gewährleistet, sowie vollständigen Umsatz der Terephthalsäure erreicht. Eine weitere Aufgabe der Erfindung ist es, ein Verfahren bereitzustellen, das in bestehenden Reaktoren für Veresterungsreaktionen durch geringfügige Umrüstungen durchgeführt werden kann.

Gegenstand der vorliegenden Erfindung ist daher ein Verfahren zur Herstellung eines Terephthalsäurediesters durch Umsetzung von Terephthalsäure mit wenigstens einem Alkohol, wobei man
a) Terephthalsäure in einem Dispergierkessel im Alkohol suspendiert, wobei man eine Vorsuspension erhält,
b) die Vorsuspension aus dem Dispergierkessel in einen Reaktor leitet und in Gegenwart eines Veresterungskatalysators umsetzt,
c) eine Reaktionssuspension aus einem zwischen dem oberen Bereich und dem unteren Bereich des Reaktors gelegenen Bereich abzieht, die abgezogene Reaktionssuspension aufteilt, einen ersten Strom der Reaktionssuspension in den oberen Bereich des Reaktors zurückführt und einen zweiten Strom der Reaktionssuspension in den unteren Bereich des Reaktors einleitet und die Reaktionssuspension so durchmischt,
d) wobei man den abgezogenen Strom und/oder den ersten Strom durch einen außerhalb des Reaktors gelegenen Wärmetauscher leitet und erwärmt; und
e) Reaktionswasser als Alkohol-Wasser-Azeotrop mit dem Brüden abdestilliert, den Brüden zumindest teilweise kondensiert, das Kondensat in eine wässrige Phase und eine organische Phase trennt und die organische Phase zumindest teilweise in das Reaktionssystem zurückführt.

Das erfindungsgemäße Verfahren kann diskontinuierlich oder kontinuierlich durchgeführt werden, wird aber vorzugsweise diskontinuierlich durchgeführt.

Das Verfahren umgeht die Probleme, die mit dem Zudosieren fester Terephthalsäure in den Reaktor verbunden sind, wie ein Verklumpen der Terephthalsäure und Verstopfen der Förderschnecke oder eines anderen Förderorgans. Das Verfahren sieht die Herstellung einer Vorsuspension in einem Dispergierkessel vor. Terephthalsäure wird nicht als Feststoff sondern in Form einer Suspension in den Reaktor dosiert.

Zur Herstellung der Vorsuspension wird pulverförmige Terephthalsäure im Dispergierkessel in einer Teilmenge des Alkohols suspendiert. Hierzu wird eine geeignete Mischvorrichtung verwendet. So kann eine Menge der Terephthalsäure mit einem Rührwerk mit Alkohol vermischt werden, alternativ können Dispergierpumpen eingesetzt werden. Dabei kann beispielsweise die gesamte Menge an Terephthalsäure in einem Schritt suspendiert werden oder im Verlauf des Verfahrens die Terephthalsäure portionsweise suspendiert werden. Für die portionsweise Suspendierung kann Terephthalsäure z.B. mit Hilfe einer Förderschnecke in den Dispergierkessel dosiert werden.

Die Vermischung kann aber auch in einer geschlossenen Kammer durch die Zusammenwirkung eines sich drehenden Rotors und eines Stators erfolgen, wobei kontinuierlich jeweils nur eine inkrementelle Menge der Komponenten miteinander vermischt wird, und die Suspension danach aus der Kammer austritt.

Als Alkohol zur Bereitung der Vorsuspension kann Frischalkohol und/oder Rückalkohol verwendet werden, d.h. die organische Phase, die nach Kondensation des Brüdens und Phasentrennung des Kondensats erhalten wird.

Der Dispergierkessel besteht meist aus metallischen Werkstoffen, wobei Edelstahl bevorzugt ist. Der Dispergierkessel kann gasseitig mit dem Reaktor verbunden sein.

Unter Verwendung einer Pumpe oder durch Gravitation wird die Vorsuspension in den Reaktor geleitet. Als Pumpen sind prinzipiell alle dem Fachmann bekannten Förderpumpen einsetzbar, die dieser unter Berücksichtigung der Eigenschaften der zu fördernden Vorsuspension als geeignet ansieht. Bevorzugt ist als Förderpumpe eine Kreisel-, Kolben-, Schnecken-, Impeller- oder Schlauchpumpe einsetzbar. Die Zudosierung der Vorsuspension in den Reaktor kann portionsweise oder kontinuierlich erfolgen. Vorzugsweise erfolgt die Zudosierung kontinuierlich. Die Vorsuspension kann prinzipiell an jeder Stelle des Reaktors eindosiert werden, vorzugsweise jedoch wird die Vorsuspension im oberen Bereich des Reaktors zugegeben, insbesondere oberhalb des Flüssigkeitsspiegels im Reaktor. Auf diese Weise kann Rückströmung entgegen der Eindosierrichtung weitestgehend verhindert werden.

Beim Reaktor kann es sich um einen beliebigen Reaktor handeln, der zur Durchführung von chemischen Umsetzungen in flüssiger Phase geeignet ist. Als Reaktoren sind nicht rückvermischte Reaktoren, wie Rohrreaktoren oder mit Einbauten versehene Verweilzeitbehälter, vorzugsweise aber rückvermischte Reaktoren, wie Rührkessel, Schlaufenreaktoren, Strahlschlaufenreaktoren oder Strahldüsenreaktoren geeignet. Gegebenenfalls können auch mehrere Reaktoren in einer mehrstufigen Apparatur zusammengefasst werden. Solche Reaktoren sind zum Beispiel Schlaufenreaktoren mit eingebauten Siebböden, kaskadierte Behälter, Rohrreaktoren mit Zwischeneinspeisung oder Rührkolonnen.

Vorzugsweise wird ein Rührkesselreaktor verwendet. Rührkesselreaktoren bestehen meist aus metallischen Werkstoffen, wobei Edelstahl bevorzugt ist.

Insbesondere bevorzugt ist die Verwendung von bestehenden Reaktionssystemen, die z.B. für die Veresterung von Phthalsäureanhydrid genutzt werden, und durch geringfügige Umrüstung für die Veresterung von Terephthalsäure eingesetzt werden können. Notwendige Umrüstungen betreffen insbesondere das Anbringen eines Dispergierkessels, eines seitlichen Abzugs am Reaktor und eines Stromteilers zum Aufteilen des abgezogenen Stroms der Reaktionssuspension.

Im Reaktor werden die Vorsuspension und der Veresterungskatalysator in Kontakt gebracht, wobei man eine Reaktionssuspension erhält. In einer Ausführungsform des Verfahrens wird dabei i) die Vorsuspension in den unbefüllten Reaktor geleitet, ii) die Vorsuspension zum Sieden erhitzt und iii) der Veresterungskatalysator zugegeben. Gegebenenfalls kann die Reihenfolge der Schritte ii) und iii) umgekehrt werden.

In einer bevorzugten Ausführungsform des Verfahrens wird jedoch der Veresterungskatalysator in einer Teilmenge Alkohol, z.B. 15-50% der Gesamtmenge des Alkohols, vorzugsweise 25-40%, im Reaktor vorgelegt. Die Katalysator/Alkohol-Mischung kann erst zum Sieden erhitzt werden und danach die Zudosierung der Vorsuspension gestartet werden. Alternativ wird die Vorsuspension zur Katalysator/Alkohol-Mischung gegeben und danach erhitzt. Gegebenenfalls können das Erhitzen der Katalysator/Alkohol-Mischung und die Zudosierung der Vorsuspension parallel durchgeführt werden.

Während der Umsetzung weist die Reaktionssuspension im Reaktor eine Temperatur nahe des Siedepunkts des Reaktionsgemisches auf, beispielsweise eine Temperatur von 150 °C bis 250 °C, vorzugsweise von 185 °C bis 220 °C. Der Siedepunkt der Reaktionssuspension ist abhängig vom Verhältnis von Terephthalsäurediester zu Alkohol und steigt im Verlauf der Reaktion an.

In einem Bereich zwischen dem oberen und unteren Bereich des Reaktors zieht man einen Strom der Reaktionssuspension aus dem Reaktor ab. Die Abzugsstelle ist vorzugsweise so gewählt, dass sich bei einem Ausfall der Umwälzpumpe suspendierte Terephthalsäure unterhalb des Abzuges sammelt. Der abgezogene Strom der Reaktionssuspension wird, z. B. mittels eines regelbaren Stromteilers, aufgeteilt in einen ersten Strom und einen zweiten Strom. Der Strom der Reaktionssuspension, der aus dem Reaktor abgezogen wird, wird im Allgemeinen in einem Verhältnis von 1 : 10 bis 10 : 1 in den ersten und den zweiten Strom aufgeteilt.

Als Pumpe sind prinzipiell alle dem Fachmann bekannten Förderpumpen, die dieser zur Durchführung des erfindungsgemäßen Verfahrens unter Berücksichtigung der Eigenschaften der zu fördernden Reaktionssuspension als geeignet ansieht, einsetzbar. Bevorzugt ist als Förderpumpe eine Kreisel-, Kolben-, Schnecken-, Impeller- oder Schlauchpumpe einsetzbar. Ganz besonders bevorzugt wird eine Axial- oder Radial-Kreiselpumpe.

Durch das Anbringen des Abzugs in einem Bereich wie oben definiert kann das Absetzen von Terephthalsäure in der Umwälzpumpe unterbunden werden. Bei einer Betriebsstörung, z. B. dem Ausfallen der Umwälzpumpe, wird dadurch das erneute Anfahren der Umwälzpumpe erleichtert.

Der Wärmeeintrag in das Reaktionssystem erfolgt erfindungsgemäß dadurch, dass der aus dem Reaktor abgezogene Strom, bevor dieser aufgeteilt wird, und/oder der erste Strom durch einen außerhalb des Reaktors gelegenen Wärmetauscher geleitet und erwärmt wird.

In einer Ausführungsform wird der aus dem Reaktor abgezogene Strom der Reaktionssuspension, bevor dieser aufgeteilt wird, durch einen außerhalb des Reaktors gelegenen Wärmetauscher geleitet und erwärmt wird. Die erwärmte Reaktionssuspension wird aufgeteilt, ein erster Strom der Reaktionssuspension wird in den oberen Bereich des Reaktors zurückgeführt und ein zweiter Strom der Reaktionssuspension in den unteren Bereich des Reaktors eingeleitet und die Reaktionssuspension so durchmischt. Sowohl der erste als auch der zweite Strom tragen zur Erwärmung des Reaktorinhalts bei; der zweite Strom trägt zur Durchmischung bei.

In einer Ausführungsform wird der aus dem Reaktor abgezogene Strom der Reaktionssuspension aufgeteilt, ein erster Strom der Reaktionssuspension wird durch einen außerhalb des Reaktors gelegenen Wärmetauscher geleitet und erwärmt und in den oberen Bereich des Reaktors zurückgeführt. Ein zweiter Strom der Reaktionssuspension wird in den unteren Bereich des Reaktors eingeleitet und die Reaktionssuspension so durchmischt.

Die Rückführung des ersten Stroms der Reaktionssuspension in den Reaktor erfolgt im oberen Bereich des Reaktors, beispielsweise auf Höhe des Flüssigkeitsspiegels der Reaktionssuspension oder im Bereich von der Höhe des Flüssigkeitsspiegels der Reaktionssuspension bis 30% darunter.

Der Volumenstrom der abgezogenen Reaktionssuspension ist beispielsweise so gewählt, dass eine Umwälzung des kompletten Reaktorinhalts in einem Zeitraum von 1 bis 60 Minuten, vorzugsweise 1 bis 10 Minuten erfolgt. Durch die konstante Umwälzung des Reaktorinhalts ist eine effektive Durchmischung der Reaktionssuspension gewährleistet.

Vorzugsweise wird der Strom der Reaktionssuspension entgegen der Richtung der Schwerkraft, also von unten nach oben, durch den außerhalb des Reaktors gelegenen Wärmetauscher geführt. Durch die vorgegebene Richtung des Stroms entgegen der Schwerkraft wird eine Sedimentation von Terephthalsäure im Wärmetauscher verhindert.

Die Reaktionssuspension wird durch das Leiten durch einen Wärmetauscher auf eine Temperatur erhitzt, bei der an der Oberfläche des Reaktionsgemisches ein hinreichend großer Brüdenstrom entsteht, um das Reaktionswasser auszutragen, beispielsweise auf eine Temperatur von 150 bis 250 °C, vorzugsweise 180 bis 220 °C.

Der zweite Strom der Reaktionssuspension wird unterhalb des Abzugs in den unteren Bereich des Reaktors eingeleitet, vorzugsweise in einem Bereich zwischen dem Reaktorboden bis 5% oberhalb des Reaktorbodens, bezogen auf die Gesamthöhe des Reaktors, und die Reaktionssuspension so durchmischt. Der zweite Strom der Reaktionssuspension, der in den Reaktor zurückdosiert wird, dient der Durchmischung der Reaktionssuspension und der Verhinderung der Sedimentation von Terephthalsäure am Boden des Reaktors. Sedimentierte Terephthalsäure steht nicht für die Veresterungsreaktion zur Verfügung. Durch die Eindosierung des zweiten Stroms unterhalb des Flüssigkeitsspiegels wird gegebenenfalls sedimentierte Terephthalsäure aufgewirbelt und kann wieder in Suspension überführt werden.

Gegebenenfalls kann die Durchmischung der Reaktionssuspension durch das Eindosieren eines Inertgases in den Reaktor, insbesondere an der tiefsten Stelle des Reaktors, und/oder den Strom der Reaktionssuspension unterstützt werden. Die Eindosierung des Inertgases trägt insbesondere bei Betriebsstörungen der Pumpe für des Abziehen der Reaktionssuspension, z.B. bei einem Ausfall der Pumpe, dazu bei, eine Sedimentation von Terephthalsäure am Reaktorboden und/oder in Rohrleitungen zu verhindern. Vorzugsweise erfolgt das Eindosieren des Inertgases an der Saugseite der Pumpe. Alternativ kann die Eindosierung an der Druckseite der Pumpe erfolgen. Dies ermöglicht die Erhaltung des Umlaufs durch den Wärmetauscher. Inertgase sind alle Gase, die unter den Reaktionsbedingungen keine Reaktivität mit den Bestandteilen der Reaktionssuspension aufweisen, insbesondere Stickstoff oder Argon. Vorzugsweise dosiert man das Inertgas in einer Menge von 0,01 bis 5 Volumeneinheiten des Inertgases pro Volumeneinheit der Reaktionssuspension pro Stunde ein.

Während der Reaktion wird ein Alkohol-Wasser-Azeotrop mit dem Brüden abdestilliert, der Brüden zumindest teilweise kondensiert, das Kondensat in eine wässrige Phase und eine organische Phase getrennt und die organische Phase zumindest teilweise in den Reaktor zurückgeführt.

Zur Kondensation bzw. partiellen Kondensation des Brüden können alle geeigneten Kondensatoren verwendet werden. Diese können mit beliebigen Kühlmedien gekühlt werden. Kondensatoren mit Luftkühlung und/oder Wasserkühlung sind bevorzugt, wobei die Luftkühlung besonders bevorzugt ist.

Das erhaltene Kondensat wird einer Phasentrennung in eine wässrige Phase und eine organische Phase unterzogen. Üblicherweise wird das Kondensat hierzu in einen Phasenscheider (Dekanter) geleitet, wo es durch mechanisches Absetzen in zwei Phasen zerfällt, die getrennt abgezogen werden können. Die wässrige Phase wird abgetrennt und kann, gegebenenfalls nach Aufarbeitung, verworfen oder als Strippwasser bei der Nachbehandlung des Esters verwendet werden.

Man führt die organische Phase über eine Kolonne (so genannte Rückalkohol-Kolonne) in den Reaktor zurück, in der man der rückgeführten organischen Phase zumindest einen Teil des Brüden entgegenführt. Bei der Rückalkohol-Kolonne kann es sich beispielsweise um eine Bodenkolonne, Packungskolonne oder Füllkörperkolonne handeln. Eine geringe Trennstufenzahl ist im Allgemeinen ausreichend. Geeignet ist z. B. eine Kolonne mit 2 bis 10 theoretischen Trennstufen. Vorzugsweise ist die Kolonne am Kopf des Reaktors aufgesetzt, also direkt mit dem Reaktor verbunden. Zweckmäßigerweise führt man die organische Phase am Kopf oder im oberen Bereich in die Rückalkohol-Kolonne ein. Das ablaufende Kondensat der Rückalkohol-Kolonne gelangt in den Reaktor zurück. Die Rückführung der organischen Phase über die Rückalkohol-Kolonne weist den Vorteil auf, dass die rückgeführte organische Phase vorerwärmt und von Wasserspuren befreit wird, die nach der Phasentrennung in der organischen Phase verblieben sind bzw. gemäß ihrer thermodynamischen Löslichkeit in der organischen Phase gelöst sind. Der Wassergehalt in der rückgeführten organischen Phase beträgt weniger als die maximale Löslichkeit von Wasser im Alkohol, vorzugsweise weniger als 3 Gew.-%, insbesondere weniger als 0,5 Gew.-%.

Im erfindungsgemäßen Verfahren werden bevorzugt lineare, verzweigte oder cyclische aliphatische Alkohole mit 4 bis 18 C-Atomen, insbesondere 8 bis 14 C-Atomen, oder aromatische Alkohole eingesetzt. Die Alkohole sind Monoole und/oder Polyole und können tertiär, sekundär oder primär sein.

Die eingesetzten Alkohole können aus verschiedenen Quellen stammen. Geeignete Einsatzstoffe sind beispielsweise Fettalkohole, Alkohole aus dem Alfolprozess oder Alkohole oder Alkoholgemische, die durch Hydrierung von gesättigten oder ungesättigten Aldehyden gewonnen wurden, insbesondere solchen, deren Synthese einen Hydroformylierungsschritt einschließt.

Alkohole, die im erfindungsgemäßen Verfahren eingesetzt werden, sind beispielsweise n-Butanol, Isobutanol, Pentanole, Hexanole, Heptanole, Octanole, wie n-Octanol, 2-Ethylhexanol, Nonanole, Decylalkohole oder Tridecanole hergestellt durch Hydroformylierung oder Aldolkondensation und anschließende Hydrierung. Die Alkohole können als reine Verbindung, als Gemisch isomerer Verbindungen oder als Gemisch von Verbindungen mit unterschiedlicher C-Zahl eingesetzt werden. Ein Beispiel eines derartigen Alkoholgemisches ist ein C₉/C₁₁-Alkoholgemisch.

Aromatische Alkohole, die im erfindungsgemäßen Verfahren eingesetzt werden können, sind beispielsweise Phenol, Benzylalkohol, 1-Naphtol, 2-Naphtol, 1,2-Dihydroxybenzol, 1,3-Dihydroxybenzol, 1,4-Dihydroxybenzol, 1,4-Naphthohydrochinon, 2,4,6-Trinitrophenol, primärer Phenylethylalkohol, sekundärer Phenylethylalkohol, Phenylpropylalkohol, o-Tolylalkohol, p-Tolylalkohol, Cuminalkohol, p-Nitrophenol, m-, o- oder p-Alkylphenol, z.B. m-, o- oder p-Methylphenol oder m-, o- oder p-Ethylphenol, m-, o- oder p-Halogenphenol, z.B. m-, o- oder p-Chlorphenol oder m-, o- oder p-Bromphenol. Weiterhin können p-Nitrobenzylalkohol, m-, o- oder p-Alkylbenzylalkohol, z.B. m-, o- oder p-Methylbenzylalkohol oder m-, o- oder p-Ethylbenzylalkohol, m-, o- oder p-Halogenbenzylalkohol, z.B. m-, o- oder p-Chlorbenzylalkohol oder m-, o- oder p-Brombenzylalkohol, 2-Ethoxyphenol, 3-Ethoxyphenol, 4-Ethoxyphenol, 2-Propoxyphenol, 3-Propoxyphenol, 4-Propoxyphenol, 2-Ethoxybenzylalkohol, 3-Ethoxybenzylalkohol, 4-Ethoxybenzylalkohol, 2-Propoxybenzylalkohol, 3-Propoxybenzylalkohol oder 4-Propoxybenzylalkohol eingesetzt werden.

Polyole, die im erfindungsgemäßen Verfahren eingesetzt werden können, sind beispielsweise 1,2-Propandiol, 1,3-Propandiol, 1,2-Butandiol, 1,3-Butandiol, 1,4-Butandiol, Neopentylglycol, 1,5-Pentandiol, 1,6-Hexandiol, 1,10-Decandiol, Diethylenglykol, 2,2,4-Trimethylpentan-1,5-diol, 2,2-Dimethylpropan-1,3-diol, 1,4-Dimethylolcyclohexan, 1,6-Dimethylolcyclohexan, Glycerin, Trimethylolpropan, Erythrit, Pentaerythrit und Sorbit.

Besonders bevorzugte Alkohohole sind 2-Ethylhexanol, 2-Propylheptanol, Isononanol-Isomerengemische, Decanol-Isomerengemische und C₉/C₁₁-Alkoholgemische.

Der umzusetzende Alkohol, der als Schleppmittel dient, kann im stöchiometrischen Überschuss eingesetzt werden. Vorzugsweise wird die Menge an eingesetztem Alkohol so gewählt, dass im Rohprodukt der Umsetzung 10 bis 35 Gew.-% Alkohol vorliegen, bezogen auf den theoretischen Vollumsatz der Terephthalsäure.

Die erfindungsgemäße Veresterung wird in Gegenwart eines Veresterungskatalysators durchgeführt.

In einer bevorzugten Ausführungsform des erfindungsgemäßen Verfahrens ist der Veresterungskatalysator im Alkohol löslich.

Geeigneterweise ist der Veresterungskatalysator unter Lewissäuren, wie Alkoholaten, Carboxylaten und Chelatverbindungen von Titan, Zirkonium, Hafnium, Zinn, Aluminium und Zink; Bortrifluorid, Bortrifluorid-Etheraten; Mineralsäuren, wie Schwefelsäure, Phosphorsäure; Sulfonsäuren, wie Methansulfonsäure und Toluolsulfonsäure, und ionischen Fluiden ausgewählt.

Geeigneterweise ist der Veresterungskatalysator unter Alkoholaten, Carboxylaten und Chelatverbindungen von Titan, Zirkonium, Hafnium, Zinn, Aluminium und Zink ausgewählt. Es eignen sich Tetraalkyltitanate, wie Tetramethyltitanat, Tetraethyltitanat, Tetra-n-propyltitanat, Tetra-isopropyltitanat, Tetra-n-butyltitanat, Tetra-isobutyltitanat, Tetrasec-butyltitanat, Tetraoctyltitanat, Tetra-(2-ethylhexyl)-titanat; Dialkyltitanate ((RO)₂TiO, worin R z.B. für iso-Propyl, n-Butyl, iso-Butyl steht), wie Isopropyl-n-butyltitanat; TitanAcetylacetonat-Chelate, wie Di-isopropoxy-bis(acetylacetonat)titanat, Di-isopropoxy-bis(ethylacetylacetonat)titanat, Di-n-butyl-bis(acetylacetonat)titanat, Di-n-butyl-bis(ethylacetoacetat)titanat, Tri-isopropoxid-bis(acetylacetonat)titanat; Zirkontetraalkylate, wie Zirkontetraethylat, Zirkontetrabutylat, Zirkontetrabutyrat, Zirkontetrapropylat, Zirkoncarboxylate, wie Zirkondiacetat; Zirkon-Acetylacetonat-Chelate, wie Zirkontetra(acetylacetonat), Tributoxyzirkonacetylacetonat, Dibutoxyzirkon(bis-acetylacetonat); Aluminiumtrisalkylate, wie Aluminiumtriisopropylat, Aluminiumtrisbutylat; Aluminium-Acetylacetonat-Chelate, wie Aluminiumtris(acetylacetonat) und Aluminiumtris(ethylacetylacetonat). Insbesondere werden Isopropyl-n-butyltitanat, Tetra(isopropyl)orthotitanat oder Tetra(butyl)orthotitanat oder Mischungen davon eingesetzt.

Geeignete ionische Fluide (ionic liquids) sind z. B. Methylimidazoliumbutansulfonsäuretriflat und 1-Ethyl-3-methyl-imidazolium-hydrogensulfat.

Die Katalysatorkonzentration hängt von der Art des Katalysators ab. Bei den bevorzugt eingesetzten Titanverbindungen beträgt diese 0,001 bis 1,0 mol% bezogen auf die Menge an Terephthalsäure, insbesondere von 0,01 bis 0,2 mol%.

Die Reaktionstemperaturen liegen zwischen 150 °C und 250 °C. Die optimalen Temperaturen hängen von den Einsatzstoffen, Reaktionsfortschritt und der Katalysatorkonzentration ab. Sie können für jeden Einzelfall durch Versuche leicht ermittelt werden. Höhere Temperaturen erhöhen die Reaktionsgeschwindigkeiten und begünstigen Nebenreaktionen, wie beispielsweise Olefinbildung oder Bildung farbiger Nebenprodukte. Es ist zur Entfernung des Reaktionswassers erforderlich, dass der Alkohol aus dem Reaktionsgemisch abdestillieren kann. Die gewünschte Temperatur oder der gewünschte Temperaturbereich kann durch den Druck im Reaktor eingestellt werden. Bei niedrig siedenden Alkoholen kann daher die Umsetzung bei Überdruck und bei höher siedenden Alkoholen bei vermindertem Druck durchgeführt werden. Beispielsweise wird bei der Umsetzung von Terephthalsäure mit 2-Ethylhexanol in einem Temperaturbereich von 180 °C bis 220 °C im Druckbereich von 300 mbar bis 2 bar gearbeitet.

Zweckmäßigerweise werden Reaktor und Dispergierkessel bei im Wesentlichen gleichem Druck betrieben werden, insbesondere etwa Umgebungsdruck. Gegebenenfalls können Reaktor und Dispergierkessel auch bei unterschiedlichen Drucken betrieben werden.

Vorzugsweise wird das erfindungsgemäße Verfahren durchgeführt, bis die Terephthalsäure im Wesentlichen vollständig umgesetzt ist. Die Bestimmung des Umsatzes kann über die Bestimmung der Säurezahl der Reaktionssuspension erfolgen. Die Säurezahl wird durch Neutralisation einer Probe der Reaktionssuspension mit Tetrabutylammoniumhydroxid bestimmt. Über die, bei der Neutralisation verbrauchte, Masse an Tetrabutylammoniumhydroxid kann die Stoffmenge der verbrauchten Tetrabutylammoniumhydroxid bestimmt werden und über stöchiometrische Betrachtungen die Stoffmenge an freien Säuregruppen nicht umgesetzter Terephthalsäure. Ausgehend von der bekannten Stoffmenge der eingesetzten Terephthalsäure kann so der Umsatz ermittelt werden. Zusätzliche Möglichkeiten zur Bestimmung des Umsatzes stellen HPLC-Messungen und die Messung der Trübung der Reaktionssuspension durch in-line-Trübungsmessungen dar. Im erfindungsgemäßen Verfahren wird vorzugsweise ein Umsatz größer als 99% erreicht.

Nach Beendigung der Reaktion enthält das Reaktionsgemisch, das im Wesentlichen aus dem gewünschten Ester und überschüssigem Alkohol besteht, neben dem Katalysator und/oder dessen Folgeprodukte geringe Mengen an Estercarbonsäure(n) und/oder nicht umgesetzter Carbonsäure.

Zur Aufarbeitung dieser Esterrohgemische wird das rohe Di-(C₄-C₁₈-alkyl)terephthalat mit einer wässrigen Base versetzt, aus dem erhaltenen Gemisch Wasser abdampft, die erhaltene flüssige Phase unter Bildung einer Wasser-in-ÖI-Emulsion mit Wasser versetzt, aus der Emulsion Wasser abdestilliert und das Di-(C₄-C₁₈-alkyl)terephthalat filtriert.

Zuerst wird der Veresterungskatalysator durch Zugabe einer wässrigen Base desaktiviert und ausgefällt. Gleichzeitig werden die bei der Veresterungsreaktion nicht umgesetzte Säure bzw. Partialester der Säure in Salze überführt.

Die Zugabe der wässrigen Base kann auf beliebige geeignete Weise erfolgen. Sie erfolgt vorzugsweise unterhalb der Flüssigkeitsoberfläche des rohen Esters. Hierfür eignen sich z. B. Lanzen oder Düsen, die an einem Behälterboden oder der Behälterwand vorgesehen sind. Das Gemisch wird dann intensiv durchmischt, z. B. mittels Rührer oder einer Umwälzpumpe.

Die zugegebene Menge wässrige Base ist so bemessen, dass sie zur vollständigen Neutralisation der sauren Komponenten des rohen Esters ausreicht. In der Praxis wird ein mehr oder weniger großer Überschuss an Base eingesetzt. Die Gesamtmenge der sauren Komponenten des rohen Esters wird zweckmäßig durch die Säurezahl (in mg KOH/g) erfasst. Vorzugsweise bringt man mit der wässrigen Base 100 bis 300% Neutralisationsäquivalente ein, bezogen auf die Säurezahl des rohen Esters, insbesondere 130 bis 220%. Unter Neutralisationsäquivalent wird dabei die Menge Base verstanden, die die gleiche Zahl Protonen binden kann, wie 1 mg KOH. Mit anderen Worten verwendet man einen Basenüberschuss von bis zu 200%, vorzugsweise 30 bis 120%.

Als wässrige Base kommen Lösungen von Hydroxiden, Carbonaten, Hydrogencarbonaten von Alkalimetallen und Erdalkalimetallen in Betracht. Wässrige Alkalimetallhydroxidlösungen sind im Allgemeinen bevorzugt. Wässrige Natriumhydroxidlösung ist aufgrund ihrer leichten Verfügbarkeit besonders bevorzugt.

Die Konzentration der wässrigen Base ist an sich nicht kritisch, jedoch kann es beim Einsatz konzentrierter Alkalilösungen an der Einleitstelle der Base zur Hydrolyse der Ester kommen. Andererseits soll die Konzentration der wässrigen Base nicht zu niedrig sein, da das mit der wässrigen Base eingebrachte Wasser im nachfolgenden Schritt wieder entfernt werden muss. Daher sind wässrige Basen mäßiger bis geringer Konzentration bevorzugt, z. B. solche einer Konzentration von 0,5 bis 25 Gew.-%, insbesondere 1 bis 10 Gew.-%. Wässrige Natriumhydroxidlösung mit einer Konzentration von 1 bis 5 Gew.-% ist besonders bevorzugt.

Oft liegt der ausgefallene Feststoff, der im Wesentlichen aus Katalysator-Zersetzungsprodukten und Salzen von nicht umgesetzter Säure bzw. Partialestern mehrbasiger Säuren besteht, in fein verteilter, schwer filtrierbarer Form vor. Zweckmäßigerweise werden die feinen Teilchen zu größeren, leicht abtrennbaren Teilchen agglomeriert.

Hierzu versetzt man die flüssige Phase unter Bildung einer Wasser-in-ÖI-Emulsion mit Wasser. Das Wasser wird als disperse Phase in Form feiner Tröpfchen in der flüssigen organischen Phase verteilt. Die feinen Feststoffteilchen wandern an die Grenzfläche zwischen Wassertröpfchen und umgebender organischer Phase. Bei der nachfolgenden Verdampfung des Wassers agglomerieren die feinen Teilchen und bilden grobe, gut abtrennbare Teilchen.

Damit sich eine eigene Wasserphase ausbildet, muss die zugegebene Wassermenge größer sein, als der Löslichkeit von Wasser in der organischen Phase entspricht. Die Wasserlöslichkeit in der organischen Phase hängt unter anderem vom Gehalt an nicht umgesetzten Alkohol ab, da der Alkohol als Lösungsvermittler wirkt. Je höher der Alkoholgehalt, umso mehr Wasser muss zur Emulsionsbildung zugesetzt werden. Bei üblichen Restalkoholgehalten von 20 bis 30 Gew.-% sind im Allgemeinen Mengen von 20 bis 80 g Wasser, vorzugsweise 30 bis 60 g, bezogen auf 1 kg rohen Ester, geeignet.

Die Wasserphase wird mit einem geeigneten Rührer oder Homogenisator oder durch Umpumpen der Emulsion unter Verwendung einer Umwälzpumpe in feine Tröpfchen zerteilt. Die erzeugten Wassertröpfchen weisen vorzugsweise eine mittlere Tröpfchengröße von weniger als 1000 µ m auf. Als Rührer mit hoher spezifischer Rührleistung eignen sich beispielsweise Scheibenrührer. Alternativ kann man besonders bei kontinuierlicher Verfahrensführung eine Mischdüse verwenden, bei der über ein Dispergierventil Wasser direkt in den Rohesterstrom zugegeben wird.

Die Emulsionsbildung erfolgt zweckmäßigerweise bei etwa Normaldruck.

Aus der so erzeugten Emulsion wird im nächsten Schritt das Wasser wieder abdestilliert.

Nach dieser Behandlung liegt der Feststoff in gut filtrierbarer Form vor; es schlägt kein Feinanteil bei der Filtration durch. Zur Filtration des Esters eignen sich alle geeigneten Filter wie Kammerfilterpressen, Bandfilter, Kerzenfilter oder Tellerfilter. Zur kontinuierlichen Verfahrensführung eignen sich besonders Tellerfilter mit Zentrifugalkuchenabwurf. Der abgetrennte Feststoff wird verworfen.

Nach der Filtration kann der Ester verschiedenen Nachbehandlungen unterzogen werden, wie einer Dampfstrippung oder dergleichen.

Die Erfindung wird durch die beigefügte Figuren näher erläutert.

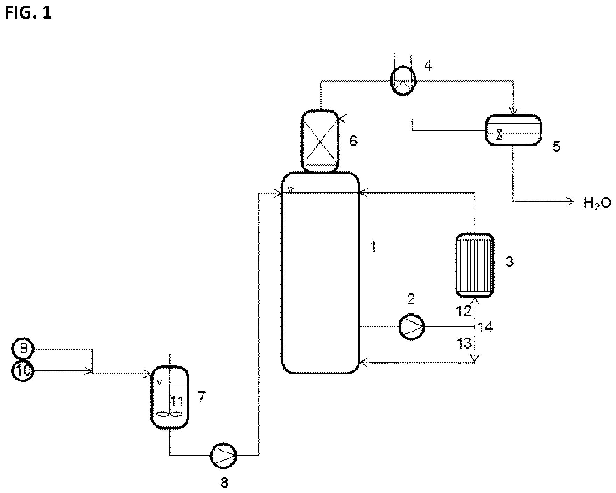

Figur 1 zeigt eine zur Durchführung des erfindungsgemäßen Verfahrens geeignete Anlage.

Gemäß Figur 1 werden in einen Dispergierkessel 7 Alkohol aus dem Reservoir 9 und Terephthalsäure aus dem Reservoir 10 zudosiert und unter Verwendung eines Rührers 11 zur Vorsuspension vermischt. Die Vorsuspension wird mit Hilfe einer Pumpe 8 in den oberen Bereich des Reaktors 1 geleitet. In dem Reaktor 1 befinden sich eine weitere Teilmenge des Alkohols und der Veresterungskatalysator. An einer Stelle des Reaktors 1 zwischen dem oberen und unteren Bereich des Reaktors wird die Reaktionssuspension unter Verwendung einer Pumpe 2 abgezogen. Die abgezogene Reaktionssuspension wir an einer Zweiwegevorrichtung 14 in einer ersten Strom 12 und einen zweiten Strom 13 aufgeteilt. Der Strom 12 wird durch einen außerhalb des Reaktors gelegenen Wärmetauscher 3 geführt. Die im Wärmetauscher 3 erwärmte Reaktionssuspension wird im oberen Bereich des Reaktors 1 wieder in diesen zurückgeführt. Der Strom 13 wird im unteren Bereich des Reaktors in den Reaktor zurückgeführt. Der Brüden tritt durch die Kolonne 6 und wird zumindest teilweise im Kondensator 4 kondensiert. Im Phasenscheider 5 wird das Kondensat in eine wässrige und eine organische Phase getrennt. Die wässrige Phase wird verworfen, die organische Phase über die Kolonne 6 in den Reaktor zurückgeführt.

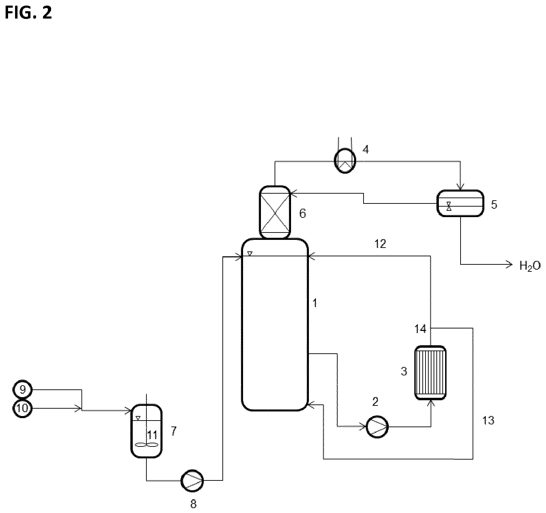

Figur 2 zeigt eine weitere zur Durchführung des erfindungsgemäßen Verfahrens geeignete Anlage.

In der Figur 2 tragen gleiche Elemente gleicher Funktionsweise die gleichen Bezugszeichen wie in Figur 1. Abweichend von Figur 1 wird der gesamte von der Pumpe 2 abgezogene Strom der Reaktionssuspension durch den Wärmetauscher 3 geführt. Die erwärmte Reaktionssuspension wird an einer Zweiwegevorrichtung 14 in einen ersten Strom 12 und einen zweiten Strom 13 aufgeteilt. Der Strom 12 wird im oberen Bereich des Reaktors 1 wieder in diesen zurückgeführt. Der Strom 13 wird im unteren Bereich des Reaktors in den Reaktor zurückgeführt.

## Patentansprüche

1. Verfahren zur Herstellung eines Terephthalsäurediesters durch Umsetzung von Terephthalsäure mit wenigstens einem Alkohol, wobei man
a) Terephthalsäure in einem Dispergierkessel im Alkohol suspendiert, wobei man eine Vorsuspension erhält,
b) die Vorsuspension aus dem Dispergierkessel in einen Reaktor leitet und in Gegenwart eines Veresterungskatalysators umsetzt,
c) eine Reaktionssuspension aus einem zwischen dem oberen Bereich und dem unteren Bereich des Reaktors gelegenen Bereich abzieht, die abgezogene Reaktionssuspension aufteilt, einen ersten Strom der Reaktionssuspension in den oberen Bereich des Reaktors zurückführt und einen zweiten Strom der Reaktionssuspension in den unteren Bereich des Reaktors einleitet und die Reaktionssuspension so durchmischt,
d) wobei man den abgezogenen Strom und/oder den ersten Strom durch einen außerhalb des Reaktors gelegenen Wärmetauscher leitet und erwärmt; und
e) Reaktionswasser als Alkohol-Wasser-Azeotrop mit dem Brüden abdestilliert, den Brüden zumindest teilweise kondensiert, das Kondensat in eine wässrige Phase und eine organische Phase trennt und die organische Phase zumindest teilweise in das Reaktionssystem zurückführt.

2. Verfahren nach Anspruch 1, wobei die zurückgeführte organische Phase einen Wassergehalt aufweist, der geringer ist als die Löslichkeit von Wasser im Alkohol.

3. Verfahren nach Anspruch 1 oder 2, wobei die in den Reaktor geleitete organische Phase einen Wassergehalt von weniger als 3 Gew.-% aufweist.

4. Verfahren nach einem der vorhergehenden Ansprüche, wobei der Veresterungskatalysator unter Lewissäuren, Mineralsäuren, Sulfonsäuren und ionischen Fluiden ausgewählt ist.

5. Verfahren nach Anspruch 4, wobei der Veresterungskatalysator unter Alkoholaten, Carboxylaten und Chelatverbindungen von Titan, Zirconium, Hafnium, Zinn, Aluminium und Zink; Bortrifluorid, Bortrifluorid-Etheraten; Schwefelsäure, Phosphorsäure; Methansulfonsäure und Toluolsulfonsäure ausgewählt ist.

6. Verfahren nach Anspruch 1 oder 2, wobei der Veresterungskatalysator unter sauren Ionentauschern, Zeolithen, Oxiden und/oder Hydroxiden von Magnesium, Aluminium, Zink, Titan, Silicium, Zinn, Blei, Antimon, Bismuth, Molybdän und Mangan ausgewählt ist.

7. Verfahren nach einem der vorhergehenden Ansprüche, wobei der Veresterungskatalysator im Alkohol löslich ist.

8. Verfahren nach einem der vorhergehenden Ansprüche, wobei der Alkohol unter linearen, verzweigten oder cyclischen aliphatischen C₄-C₁₈-Alkoholen oder aromatischen Alkoholen ausgewählt ist.

9. Verfahren nach einem der vorhergehenden Ansprüche, wobei das Verfahren kontinuierlich oder diskontinuierlich durchgeführt wird.

10. Verfahren nach einem der vorhergehenden Ansprüche, wobei man die Umsetzung im Reaktor bei einer Temperatur von 100 bis 250 °C durchführt.

11. Verfahren nach einem der vorhergehenden Ansprüche, wobei man den Alkohol in einem solchen stöchiometrischen Überschuss einsetzt, dass das rohe Veresterungsprodukt 15 bis 35 Gew.-% Alkohol enthält.

12. Verfahren nach einem der vorhergehenden Ansprüche, wobei man ein Inertgas zur Fluidisierung in den Reaktor und/oder den Strom der Reaktionssuspension eindosiert.

13. Verfahren nach einem der vorhergehenden Ansprüche, wobei man zur Aufarbeitung den rohen Terephthalsäurediester mit einer wässrigen Base versetzt, aus dem erhaltenen Gemisch Wasser abdampft, die erhaltene flüssige Phase unter Bildung einer Wasser-in-ÖI-Emulsion mit Wasser versetzt, aus der Emulsion Wasser abdestilliert und den Terephthalsäurediester filtriert.

14. Verfahren nach einem der vorhergehenden Ansprüche, wobei der abgezogene Volumenstrom der Reaktionssuspension so gewählt ist, dass eine vollständige Umwälzung des Reaktorinhalts in einem Zeitraum von 1 bis 10 Minuten erfolgt.

## Claims

1. A process for preparing a terephthalic diester by reacting terephthalic acid with at least one alcohol, wherein
a) terephthalic acid is suspended in the alcohol in a dispersing tank to obtain a preliminary suspension,
b) the preliminary suspension is passed from the dispersing tank into a reactor and converted in the presence of an esterification catalyst,
c) a reaction suspension is drawn off from a region between the upper region and the lower region of the reactor, the reaction suspension drawn off is divided, a first stream of the reaction suspension is recycled into the upper region of the reactor and a second stream of the reaction suspension is introduced into the lower region of the reactor, and the reaction suspension is thus mixed,
d) wherein the stream drawn off and/or the first stream is passed through a heat exchanger outside the reactor and heated; and
e) water of reaction is distilled off together with the vapor as alcohol-water azeotrope, the vapor is at least partly condensed, the condensate is separated into an aqueous phase and an organic phase and the organic phase is at least partly recycled into the reaction system.

2. The process according to claim 1, wherein the organic phase recycled has a water content lower than the solubility of water in the alcohol.

3. The process according to claim 1 or 2, wherein the organic phase passed into the reactor has a water content of less than 3% by weight.

4. The process according to any of the preceding claims, wherein the esterification catalyst is selected from Lewis acids, mineral acids, sulfonic acids and ionic fluids.

5. The process according to claim 4, wherein the esterification catalyst is selected from alkoxides, carboxylates and chelate compounds of titanium, zirconium, hafnium, tin, aluminum and zinc; boron trifluoride, boron trifluoride etherates; sulfuric acid, phosphoric acid; methanesulfonic acid and toluenesulfonic acid.

6. The process according to claim 1 or 2, wherein the esterification catalyst is selected from acidic ion exchangers, zeolites, oxides and/or hydroxides of magnesium, aluminum, zinc, titanium, silicon, tin, lead, antimony, bismuth, molybdenum and manganese.

7. The process according to any of the preceding claims, wherein the esterification catalyst is soluble in the alcohol.

8. The process according to any of the preceding claims, wherein the alcohol is selected from linear, branched and cyclic aliphatic C₄-C₁₈ alcohols and aromatic alcohols.

9. The process according to any of the preceding claims, which is performed continuously or batchwise.

10. The process according to any of the preceding claims, wherein the reaction in the reactor is conducted at a temperature of 100 to 250°C.

11. The process according to any of the preceding claims, wherein the alcohol is used in such a stoichiometric excess that the crude esterification product comprises 15% to 35% by weight of alcohol.

12. The process according to any of the preceding claims, wherein an inert gas is metered into the reactor and/or the stream of the reaction suspension for fluidization.

13. The process according to any of the preceding claims, wherein the crude terephthalic diester is worked up by admixing with an aqueous base, evaporating water out of the mixture obtained, admixing the liquid phase obtained with water to form a water-in-oil emulsion, distilling water out of the emulsion and filtering the terephthalic diester.

14. The process according to any of the preceding claims, wherein the volume flow rate of the reaction suspension drawn off is chosen such that the reactor contents are circulated completely within a period of 1 to 10 minutes.

## Revendications

1. Procédé de fabrication d'un diester de l'acide téréphtalique par mise en réaction d'acide téréphtalique avec au moins un alcool, selon lequel
a) l'acide téréphtalique est suspendu dans l'alcool dans une cuve de dispersion, une pré-suspension étant obtenue,
b) la pré-suspension est acheminée depuis la cuve de dispersion dans un réacteur et mise en réaction en présence d'un catalyseur d'estérification,
c) une suspension de réaction est soutirée à partir d'une zone située entre la zone supérieure et la zone inférieure du réacteur, la suspension de réaction soutirée est divisée, un premier courant de la suspension de réaction est recyclé dans la zone supérieure du réacteur et un deuxième courant de la suspension de réaction est introduit dans la zone inférieure du réacteur, et la suspension de réaction est ainsi mélangée,
d) le courant soutiré et/ou le premier courant étant acheminés au travers d'un échangeur de chaleur situé à l'extérieur du réacteur et chauffés ; et
e) l'eau de réaction est éliminée par distillation sous la forme d'un azéotrope alcool-eau avec les vapeurs, les vapeurs sont au moins partiellement condensées, le condensat est séparé en une phase aqueuse et une phase organique, et la phase organique est au moins partiellement recyclée dans le système de réaction.

2. Procédé selon la revendication 1, dans lequel la phase organique recyclée présente une teneur en eau qui est inférieure à la solubilité de l'eau dans l'alcool.

3. Procédé selon la revendication 1 ou 2, dans lequel la phase organique introduite dans le réacteur présente une teneur en eau inférieure à 3 % en poids.

4. Procédé selon l'une quelconque des revendications précédentes, dans lequel le catalyseur d'estérification est choisi parmi les acides de Lewis, les acides minéraux, les acides sulfoniques et les fluides ioniques.

5. Procédé selon la revendication 4, dans lequel le catalyseur d'estérification est choisi parmi les alcoolates, les carboxylates et les composés chélatés de titane, zirconium, hafnium, étain, aluminium et zinc ; le trifluorure de bore, les éthérates de trifluorure de bore ; l'acide sulfurique, l'acide phosphorique ; l'acide méthane-sulfonique et l'acide toluène-sulfonique.

6. Procédé selon la revendication 1 ou 2, dans lequel le catalyseur d'estérification est choisi parmi les échangeurs d'ions acides, les zéolithes, les oxydes et/ou les hydroxydes de magnésium, aluminium, zinc, titane, silicium, étain, plomb, antimoine, bismuth, molybdène et manganèse.

7. Procédé selon l'une quelconque des revendications précédentes, dans lequel le catalyseur d'estérification est soluble dans l'alcool.

8. Procédé selon l'une quelconque des revendications précédentes, dans lequel l'alcool est choisi parmi les alcools en C₄-C₁₈ aliphatiques linéaires, ramifiés ou cycliques ou les alcools aromatiques.

9. Procédé selon l'une quelconque des revendications précédentes, dans lequel le procédé est réalisé de manière continue ou discontinue.

10. Procédé selon l'une quelconque des revendications précédentes, dans lequel la réaction est réalisée dans le réacteur à une température de 100 à 250 °C.

11. Procédé selon l'une quelconque des revendications précédentes, dans lequel l'alcool est utilisé en un excès stoechiométrique tel que le produit d'estérification brut contienne 15 à 35 % en poids d'alcool.

12. Procédé selon l'une quelconque des revendications précédentes, dans lequel un gaz inerte est introduit pour la fluidisation dans le réacteur et/ou le courant de la suspension de réaction.

13. Procédé selon l'une quelconque des revendications précédentes, dans lequel, pour le traitement, le diester de l'acide téréphtalique brut est mélangé avec une base aqueuse, l'eau est évaporée du mélange obtenu, la phase liquide obtenue est mélangée avec de l'eau pour obtenir une émulsion eau dans huile, l'eau est éliminée par distillation de l'émulsion et le diester de l'acide téréphtalique est filtré.

14. Procédé selon l'une quelconque des revendications précédentes, dans lequel le débit volumique soutiré de la suspension de réaction est choisi de telle sorte qu'une rotation complète du contenu du réacteur ait lieu en une durée de 1 à 10 minutes.
